# EUROPÄISCHE PATENTSCHRIFT

(11) **EP 1 091 659 B1**
(45) Veröffentlichungstag und Bekanntmachung des Hinweises auf die Patenterteilung: **15.05.2002**
(21) Anmeldenummer: 00922634.1
(22) Anmeldetag: 13.04.2000
(51) Int. Cl.: A23L 1/30, A61K 9/48, A61K 31/202, A61K 31/232, A61P 1/00

(54) **ORALE DARREICHUNGSFORM**
ORAL FORM OF ADMINISTRATION
FORME GALENIQUE ORALE

(30) Priorität: 30.06.1999 DE 19930030
(43) Veröffentlichungstag der Anmeldung: 18.04.2001
(73) Patentinhaber: Meduna Arzneimittel GmbH, 30916 Isernhagen (DE)
(72) Erfinder: FRAUENDORFER, Friedel, D-30938 Grossburgwedel (DE)
(74) Vertreter: Patentanwälte Hauck, Graalfs, Wehnert, Döring, Siemons
(86) Internationale Anmeldenummer: EP0003350
(87) Internationale Veröffentlichungsnummer: WO0101797

(56) Entgegenhaltungen:
- EP-A- 0 240 581
- EP-A- 0 369 445
- WO-A-93/13761
- WO-A-96/36329
- WO-A-97/04755
- DATABASE WPI Section Ch, Week 199711 Derwent Publications Ltd., London, GB; Class B04, AN 1997-112791 XP002143507 -& JP 09 000201 A (AMINO APPU KAGAKU KK), 7. Januar 1997 (1997-01-07)

## Beschreibung

Die Erfindung bezieht sich auf eine orale Darreichungsform für Nahrungsmittelsowie Nahrungsergänzungsmittel und Diätetika.

Es ist bekannt, im Bereich der Nahrungsergänzung, Diätetika und Arzneimittel Omega 3 mehrfach ungesättigte Fettsäuren anzuwenden. Zur Bereitstellung der mehrfach ungesättigten Fettsäuren werden unter anderem Fischöl, Leinsamenöl, Lebertran oder dergleichen verwendet. Es ist bekannt, diese Substanzen in Gelatinekapseln zu verbringen, um den unangenehmen Geschmack und ein Aufstoßen zu beherrschen. Um das Risiko einer raschen Oxydation und damit einer erhöhten Toxizität zu mindern, werden dem Öl antioxydative Substanzen beigemischt. Eine rasch steigende Oxydation (Ranzigwerden) bedeutet nicht nur die Entwicklung körperschädigender Radikale, sondern reduziert auch die Haltbarkeit des Produkts. Ein weiteres Problem ist das Risiko, daß mehrfach ungesättigte Fettsäuren, bevor diese den Dünndarm erreichen, bereits im Magen und Zwölffingerdarm unerwünschten Veränderungen unterzogen werden und ggf. am Ort der Resorption nicht oder nur teilweise zur Verfügung stehen.

Aus WO 90/04391 ist eine orale Darreichungsform von Omega 3 mehrfach ungesättigten Fettsäuren bekannt zur Bekämpfung von Gefäßkrankheiten. Es ist bekannt, derartige Fettsäuren in Weichgelatinekapseln darzureichen. Aus WO 96/36329 ist auch bekannt, Gelatinekapseln, die mit Omega 3 mehrfach ungesättigten Fettsäuren gefüllt sind, mit einer Beschichtung zu versehen aus einem neutralen Polyacrylat bzw. einem Polyethylacrylat oder einem Methylmethacrylat. Die Beschichtung soll verhindern, daß die Kapsel bereits im Magen die Fettsäure freisetzt.

Eine reine Gelatinekapsel verhindert weder das Risiko einer Veränderung der Struktur der mehrfach ungesättigten Fettsäuren, noch das unangenehme Aufstoßen aus dem Magen mit seinem unangenehmen Geruch.

Aus EP 2 240 581 B1 sind eine Gelatinekapsel für Arzneimittel mit gesteuerter Wirkstofffreisetzung und ein Verfahren zu deren Herstellung bekannt. In dem Verfahren wird dem Gelatinesud Xylose zugegeben und anschließend zu einer Gelatinekapsel verarbeitet. Die so hergestellten Gelatinekapseln weisen eine retardierte Freisetzung auf.

Der Erfindung liegt die Aufgabe zu Grunde, eine orale Darreichungsform für ungesättigte Fettsäuren enthaltende Nahrungs- und Nahrungsergänzungsmittel sowie Diätetika zu schaffen, mit der die ungesättigten Fettsäuren länger lagerfähig sind. Außerdem soll die Darreichungsform lebensmittelrechtlich unbedenklich sein.

Diese Aufgabe wird durch die Merkmale des Patentanspruchs 1 gelöst.

Bei der erfindungsgemäßen Darreichungsform ist die ungesättigte Fettsäure in einer Gelatinekapsel enthalten. Diese ist mit Xylose gehärtet. Mit der Härtung kann eine Kapselöffnungszeit von beispielsweise 45 min. und mehr eingestellt werden.

Während üblicherweise Fettsäuren Antioxydantien, wie Tocopherole, Ascorbylpalmitat, propyl. Gallate etc. beigemischt werden, entfällt eine derartige Behandlung bei der Erfindung, da die Xylosehärtung einen Fettverderb verhindert. Für den Fettverderb ist die Peroxidation der ungesättigten Fettsäure eine wesentliche Reaktion. Bei der erfindungsgemäßen Darreichungsform wurden überraschender Weise eine geringe Peroxidbildung und ein deutlich verzögertes Ranzigwerden festgestellt.

Die erfindungsgemäße orale Darreichungsform ermöglicht eine ungestörte Freisetzung der mehrfach ungesättigten Fettsäure nach der Magenpassage erst im Darmbereich. Ein unangenehmer Geschmack und ein Aufstoßen wird verhindert.

Xylose ist ein in der Lebensmittelindustrie an sich gebräuchlicher Hilfsstoff, der unter anderem als Abfall bei der Zelluloseproduktion entsteht und der auch als Süßungsmittel geeignet ist. Außerdem wirkt er leicht abführend.

Für die ungesättigten Fettsäuren können Omega-3 mehrfach ungesättigte Fettsäuren mit einem hohen Gehalt an Alpha-Linolensäure, bevorzugt Perillaöl, verwendet werden. Auch die Verwendung von Fischöl, Leinöl und Gamma-Linolensäure ist bevorzugt möglich.

Die erfindungsgemäße Darreichungsform ist für essentielle Fettsäuren aller Art, die anfällig für die Bildung toxischer Radikale sind, sehr gut geeignet. Für ihren Einsatz lassen sich folgende Forderungen aufstellen:
- Peroxid-Wert < 2,
- keine vorzeitige Zersetzung im Magen und Zwölffingerdarm,
- Resorption im Dünndarm.

In überraschender Weise werden sämtliche Anforderungen durch die erfindungsgemäße Darrreichungsform erfüllt.

Nach einer besonderen Ausgestaltung der Erfindung wird die Gelatinekapsel mit Perillaöl gefüllt. Perillaöl wird aus den ölhaltigen Früchten der asiatischen Pflanze Perilla frutescens gewonnen und enthält über 70 % Anteil ungesättigter Fettsäuren, vor allem α- Linolensäure.

Eine Vielzahl von wissenschaftlichen Studien hat positive Effekte bei Fettstoffwechselstörungen (metabolisches Syndrom) und entzündungshemmende Wirkung im Darmbereich (Morbus Crohn) gezeigt. Perillaöl hat darüber hinaus den Vorteil, daß es nahezu geschmacks- und geruchlos ist.

Zwei galenische Formen, nämlich Reingelatinekapseln und xylosegehärtete Kapseln jeweils mit Perillaöl, wurden bei 21° C und 45 % Luftfeuchtigkeit über einen Zeitraum von 12 Monaten auf Ihre Peroxidzahl geprüft. Der Peroxidwert der xylosegehärteten Kapsel war signifikant niedriger als der für die Reingelatinekapsel und stieg im Laufe des Beobachtungszeitraums nicht an, sondern fiel sogar.

In einer Verträglichkeitsstudie an 24 Probanden über 4 Wochen bei Einnahme von 3-6 Kapseln à 500 mg Perillaöl trat keine Übelkeit, kein Magendruck auf, noch wurden andere Auffälligkeiten beobachtet. Die Geschmacksempfindung wurde in keinem Fall beeinträchtigt.

### Beispiel:

500 mg Perillaöl-Kapsel ohne Xylosehärtung im Langzeittest:

| | 0 Monate | 3 Monate | 6 Monate | 12 Monate |
|---|---|---|---|---|
| Perillaöl / Perillaöl mg | 498,2 | 506,2 | 513,5 | 486,1 |
| Perillaöl / α-Linolensäure mg | 260 | 264,2 | 268 | 253,7 |
| Peroxidzahl | 2,3 | 2,5 | 3,1 | 3 |

500 mg Perillaöl-Kapseln mit Xylosehärtung im Langzeittest:

| | 0 Monate | 3 Monate | 6 Monate | 12 Monate |
|---|---|---|---|---|
| Perollaöl / Perillaöl mg | 498,7 | 508,1 | 513,8 | 489,2 |
| Perillaöl / α-Linolensäure mg | 260,3 | 265,2 | 268,2 | 255,5 |
| Peroxidzahl | 2,1 | 2,1 | 1,6 | 1 |

Als Verpackungsmaterial wird Blister verwendet.

Xylosehärtung kann nach den in EP 0240581 B1 angegebenen Beispielen, insbesondere dem dortigen Beispiel 3, erfolgen. Alternativ hierzu ist es möglich, die Kapseln mit einer Lösung aus Xylose, Ethanol und Wasser über einen bestimmten Zeitraum gleichmäßig zu besprühen. Während dieser Zeit werden die Kapseln erhitzt. Nach dem Auftragen der berechneten Menge Härtungslösung werden die Kapseln über einen bestimmten Zeitraum wärmebehandelt. Diese Wärmebehandlung führt dazu, daß die Aldehydfunktion der Xylose mit Gelatine reagiert und sich eine Quervernetzung bildet. Letztere bewirkt die Härtung der Gelatinekapsel. Das gebildete Produkt weist eine die Peroxidation der Fettsäure hemmende Struktur auf, die einen Zusatz von Antioxidantien unnötig macht.

## Patentansprüche

1. Orale Darreichungsform für Nahrungs- sowie Nahrungsergänzungsmittel und Diätetika umfassend mehrfach ungesättigte Fettsäuren in einer xylosegehärteten Gelatinekapsel mit einer verzögerten Kapselöffnungszeit.

2. Darreichungsform nach Anspruch 1, **gekennzeichnet, durch** Omega-3 mehrfach ungesättigte Fettsäuren mit einem hohen Gehalt an Alpha-Linolensäure.

3. Darreichungsform nach Anspruch 2, **dadurch gekennzeichnet, daß** sie Perillaöl enthält.

4. Darreichungsform nach einem der Ansprüche 1 bis 3, **dadurch gekennzeichnet, daß** die Kapselöffnungszeit auf mehr als 45 min. eingestellt ist.

5. Darreichungsform nach einem der Ansprüche 1 bis 4, zur Anwendung bei Fettstoffwechselstörungen und/oder Darmentzündungen, z. B. Morbus Crohn oder Colitis ulcerosa.

6. Darreichungsform nach Anspruch 1, **dadurch gekennzeichnet, daß** sie Fischöl, Leinöl oder Gamma-Linolensäure enthält.

## Claims

1. Oral dosage form for food and food supplement as well as dietetics comprising polyunsaturated fatty acids in a xylose-hardened gelatine capsule with a retarded release time.

2. Dosage form according to claim 1 comprising omega-3 polyunsaturated fatty acids with a high content of alpha linolenic acid.

3. Dosage form according to claim 2 comprising perilla oil.

4. Dosage form according to one of the claims 1 to 3, **characterized in that** the retarded release time of the capsule is adjusted to more than 45 minutes.

5. Dosage form according to one of the claims 1 to 4 for use against diseases of metabolism of fat and/or against intestinal inflammations, such as Morbus Crohn or colitis ulcerosa.

6. Dosage form according to claim 1 comprising fish oil, linseed oil or gamma linolenic acid.

## Revendications

1. Forme posologique orale pour produits alimentaires ainsi que de compléments alimentaires et de produits diététiques contenant des acides gras polyinsaturés dans une capsule de gélatine durcie avec de la xylose ayant un instant d'ouverture retardé.

2. Forme posologique selon la revendication 1, **caractérisée en ce qu'**elle contient des acides gras oméga 3 polyinsaturés comportant une haute teneur en acide alpha-linolénique.

3. Forme posologique selon la revendication 2, **caractérisée en ce qu'**elle contient de l'huile de périlla.

4. Forme posologique selon l'une des revendications 1 à 3, **caractérisée en ce que** l'instant d'ouverture de la capsule est retardé de plus de 45 min.

5. Forme posologique selon l'une des revendications 1 à 4, destinée à être utilisée en cas de troubles d'échange lipidiques et/ou d'inflammations intestinales, par ex. la maladie de Crohn ou la colite ulcéreuse.

6. Forme posologique selon la revendication 1, **caractérisée en ce qu'**elle contient de l'huile de poisson, de l'huile de lin ou de l'acide gamma-linolénique.
